# EUROPÄISCHE PATENTSCHRIFT

(11) **EP 0 695 744 B1**
(45) Veröffentlichungstag und Bekanntmachung des Hinweises auf die Patenterteilung: **08.10.1997**
(21) Anmeldenummer: 95111581.5
(22) Anmeldetag: 24.07.1995
(51) Int. Cl.: C07D 277/18

(54) **Verfahren zur Herstellung von Cyanimino-1,3-thiazolidinen**
Process for the preparation of cyanimino-1,3-thiazolidine
Procédé pour la préparation de la cyanimino-1,3-thiazolidine

(30) Priorität: 04.08.1994 DE 4427569
(43) Veröffentlichungstag der Anmeldung: 07.02.1996
(73) Patentinhaber: BAYER AG, 51368 Leverkusen (DE)
(72) Erfinder: Lantzsch, Reinhard, Dr., D-42115 Wuppertal (DE)

(56) Entgegenhaltungen:
- US-A- 4 616 025
- JOURNAL OF HETEROCYCLIC CHEMISTRY, Bd. 24, 1987 Seiten 275-278, WEBB ET AL. 'Diphenyl Cyancarbonimidate ...'
- CHEMISTRY AND INDUSTRY, 2.Mai 1983 Seiten 349-352, ROBINSON 'Development of ...'
- ORG. PREP. PROC. INT., Bd. 23, Nr. 6, 1991 Seiten 721-728, ZMITEK ET AL. 'The synthesis and reactions of ...'
- CHEMICAL ABSTRACTS, vol. 78, no. 5, 1973 Columbus, Ohio, US; abstract no. 29657r, NEIDLEIN ET AL. 'Reactions of dimethyl N.cyanoimidodithiocarbonate ...' Seite 494;

## Beschreibung

Die Erfindung betrifft ein neues Verfahren zur Herstellung von Cyanimino-1,3-thiazolidin.

Es ist bekannt, daß man Cyanimino-1,3-thiazolidin erhält, wenn man Dimethyl-N-cyanodithiocarbonat und Cysteamin in Ethanol am Rückfluß erhitzt (vgl. Archiv der Pharmazie 305, 731 (1972). Dieses Verfahren weist jedoch den Nachteil auf daß zwei Äquivalente Methylmercaptan abgespalten werden. Die technische Herstellung auf diesem Weg ist daher aus Toxikologie- und Umweltaspekten sehr kostspielig, da Methylmercaptan ein starkes Atemgift ist und zusätzliche Sicherheitsmaßnahmen erfoderlich macht und entweder verbrannt oder durch Oxidation mit Chlorlauge oder Wasserstoffperoxid vernichtet werden muß.

Weiter ist bekannt, daß man Cyanimino-1,3-thiazolidin erhält, wenn man Dimethyl-N-cyanimidocarbonat mit Cysteamin in wäßriger Natronlauge bei einem pH-Wert von 10-11 längere Zeit rührt (vgl. Org. Prep. Procedures Int. 23, (6), 721-728 (1991). Der Schmelzpunkt des so erhaltenen Produktes (Fp. 168-170°C) weicht aber erheblich von dem des reinen Cyanimino-1,3-thiazolidins (Fp. 154-156°C) ab, da dieses vermutlich durch Nebenprodukte verunreinigt ist.

Durch eine weitere Reinigung wurde daher die mit 48% angegebene Ausbeute noch weiter vermindert, so daß auch dieses Verfahren für eine technische Herstellung nicht geeignet ist.

Überraschenderweise wurde nun gefunden, daß man Cyanimino-1,3-thiazolidin in sehr guter Ausbeute und Reinheit erhält, wenn man Cysteaminsalze der Formel (I) 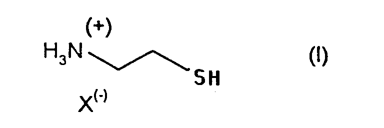 in welcher
- X: für einen Säurerest steht,
in Gegenwart eines Verdünnungsmittels, in Gegenwart von mindestens 2 Äquivalenten Base und gegebenenfalls in Gegenwart einer Schutzgasatmosphäre mit Dialkyl-N-cyanimidocarbonaten der Formel (II) 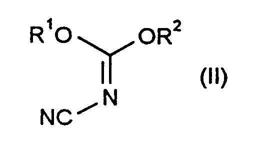 in welcher
- R¹ und R²: für Methyl, Ethyl, n-Propyl, i-Propyl, n-Butyl, i-Butyl, sec.-Butyl oder tert.-Butyl stehen,
zu dem intermediär auftretenden Zwischenprodukt der Formel (III) 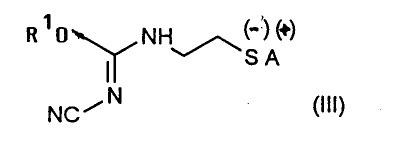 in welcher
- A⁽⁺⁾: für ein Metallionenäquivalent oder für ein Ammoniumion steht,
umsetzt und anschließend bei einem pH-Wert von 8 bis 9,5 zu Cyanimino-1,3-thiazolidin der Formel (IV) 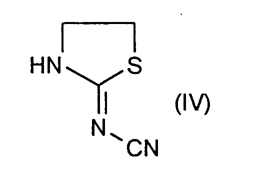 cyclisiert.

Es ist als ausgesprochen überraschend anzusehen, daß nach dem erfindungsgemäßen Verfahren das Cyanimino-1,3-thiazolidin in hoher Reinheit und Ausbeute erhalten wird, da bekannt ist, daß bei der Reaktion von Dimethyl-N-cyanimidocarbonat mit Cysteamin in Wasser bei 0°C entweder N-Cyano-N'-(2-mercaptoethyl)-carbamimidinsäuremethylester oder die daraus resultierende Disulfidverbindung entsteht [vgl. Chemistry and Industry 1983, 349-352; Org. Prep. Procedures Int. 23, (6), 721-728 (1991)]. Das neue Verfahren stellt somit eine wesentliche Verbesserung gegenüber dem Stand der Technik dar.

Für das erfindungsgemäße Verfahren werden vorzugsweise Verbindungen der Formel (I) eingesetzt, in welchen
- X: für einen Säurerest wie beispielsweise Halogen, Acetat, Sulfat oder Hydrogensulfat steht.

Für das erfindungsgemäße Verfahren werden außerdem vorzugsweise Verbindungen der Formel (II) eingesetzt, in welchen
- R¹ und R²: für Methyl oder Ethyl stehen.

In dem bei dem erfindungsgemäßen Verfahren intermediär auftretenden Zwischenprodukt der Formel (III) steht vorzugsweise
- A: für ein Natrium-, Kalium- oder Ammoniumion.

Verwendet man beispielsweise Cysteamin-Hydrochlorid, Natronlauge und Dimethyl-N-cyanimidocarbonat, so läßt sich das erfindungsgemäße Verfahren durch das folgende Formelschema beschreiben.

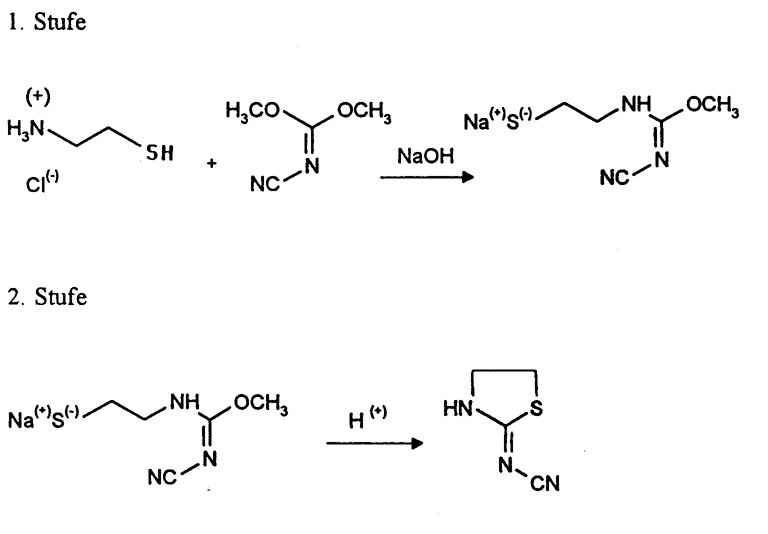

Die bei dem erfindungsgemäßen Verfahren als Ausgangsstoffe zu verwendenden Cysteaminsalze der Formel (I) und die Dialkyl-N-cyanimidocarbonate der Formel (II) sind allgemein bekannte Verbindungen der organischen Chemie.

Das erfindungsgemäße Verfahren wird vorzugsweise in Gegenwart eines Verdünnungsmittels durchgeführt. Als Verdünnungsmittel kommen alle unter den Reaktionsbedingungen inerten üblichen Losungsmittel in Betracht. Hierzu gehören beispielsweise Wasser, Alkohole, wie beispielsweise Methanol, Ethanol, Propanol oder Butanol; Nitrile, wie beispielsweise Acetonitril, Butyronitril oder Isobutyronitril oder Ether, wie beispielsweise Dimethoxyethan, Methyl-tert.-butylether oder TAME. Besonders bevorzugt wird die Reaktion in Wasser oder Wasser/Alkohol-Gemisch durchgeführt.

Die Reaktionstemperaturen können bei der Durchführung des erfindungsgemäßen Verfahrens zwischen 0°C und 70°C variiert werden.

Das erfindungsgemäße Verfahren wird so durchgeführt, daß das entsprechende Cysteaminsalz der Formel (I) in einer Mischung aus einer Base und einem der oben angegebenen Lösungsmittel vorgelegt wird und danach bei der angegebenen Temperatur portionsweise mit einem der Dialkyl-N-cyanimidocarbonate der Formel (II) versetzt wird. Durch Zusatz einer Mineralsäure, wie beispielsweise Schwefelsäure oder Salzsäure, welche gegebenenfalls auch gasförmig eingesetzt werden kann, erfolgt dann bei einem pH-Wert zwischen 8 und 9,5 die Cyclisierung. Eine umgekehrte Dosierung ist ebenfalls möglich: Das Dialkyl-N-cyanimidocarbonat wird in einem Lösungsmittel vorgelegt und eine Lösung des Cysteaminsalzes mit mindestens 2 Äquivalenten Base zudosiert. Bei dieser Verfahrensweise entsteht besonders wenig unerwünschtes Disulfid. Die Aufarbeitung kann nach üblichen Methoden erfolgen (vgl. Herstellungsbeispiel).

Als Basen kommen Alkalimetallhydroxide, wie z.B. Natrium- und Kaliumhydroxide, Erdalkalihydroxide, wie z.B. Calciumhydroxid, Alkalicarbonate und - alkoholate wie Natrium- und Kaliumcarbonat, Natrium- und Kaliummethylat bzw. -ethylat, infrage, welche im Überschuß eingesetzt werden.

Das erfindungsgemäße Verfahren wird gegebenenfalls in Gegenwart eines Inertgases durchgeführt. Als Inertgas kommen dabei Stickstoff sowie praktisch alle Edelgase, insbesondere Argon infrage.

Das nach dem erfindungsgemäßen Verfahren hergestellte Cyanimino-1,3-thiazolidin kann als Ausgangsstoff zur Herstellung von Schädlingsbekämpfungsmitteln verwendet werden (vgl. EP-A 235 725).

Die Erfindung wird durch das folgende Beispiel verdeutlicht.

### Beispiel 1

17,8 g (0,2 Mol) 45%ige Natronlauge wird unter Schutzgasatmosphäre vorgelegt, mit 30 ml Wasser verdünnt und mit 11,6 g (0,1 Mol) Cysteamin-Hydrochlorid versetzt. Die Lösung wird 15 Minuten bei 30-35°C gerührt und dann auf 0°C abgekühlt.

Nun werden portionsweise 11,4 g (0,1 Mol) Dimethyl-N-cyanimidocarbonat zudosiert und 2,5 Stunden bei 0-5°C gerührt. Der pH-Wert beträgt 12,7. Man läßt auf Raumtemperatur kommen und stellt den pH-Wert durch Salzsäurezugabe auf 9,5 ein, erwärmt auf 40°C und stellt den pH-Wert auf 9,0. Das Reaktionsgemisch wird 8 Stunden gerührt, der pH-Wert auf 6,8 gestellt und der Feststoff abgesaugt und getrocknet.

Man erhzält 10,9 g Cyanimino-1,3-thiazolidin vom Schmelzpunkt 154°C. Der Gehalt beträgt laut HPLC 95,8 %. Der Gehalt an Disulfid beträgt 1,9 %; dies entspricht einer Ausbeute von 82,1 % der Theorie.

## Patentansprüche

1. Verfahren zur Herstellung von Cyanimino-1,3-thiazolidin, dadurch gekennzeichnet, daß man bei Temperaturen zwischen 0 bis 70°C Cysteaminsalze der Formel (I) 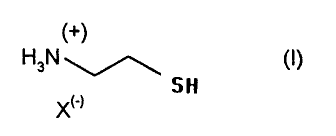 in welcher
X für einen Säurerest steht,
in Gegenwart eines Verdünnungsmittels, in Gegenwart von mindestens 2 Äquivalenten Base und gegebenenfalls in Gegenwart einer Schutzgasatmosphäre mit Dialkyl-N-cyanimidocarbonaten der Formel (II) 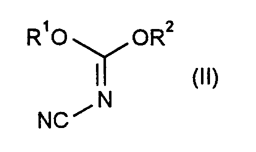 in welcher
R¹ und R² für Methyl, Ethyl, n-Propyl, i-Propyl, n-Butyl, i-Butyl, sec.-Butyl oder tert.-Butyl stehen,
zu dem intermediär auftretenden Zwischenprodukt der Formel (III) 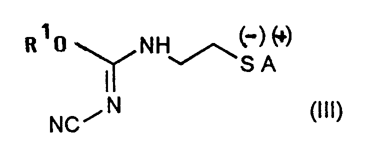 in welcher
R¹ die oben angegebene Bedeutung besitzt,
A⁽⁺⁾ für ein Metallionenäquivalent oder für ein Ammoniumion steht,
umsetzt und anschließend bei einem pH-Wert von 8 bis 9,5 zu Cyanimino-1,3-thiazolidin der Formel (IV) 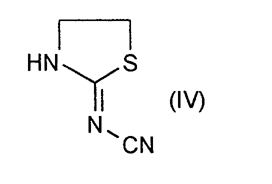 cyclisiert.

2. Verfahren gemäß Anspruch 1, dadurch gekennzeichnet, daß Verbindungen der Formel (I) eingesetzt werden, in welchen
X für einen Säurerest der Gruppe Halogen, Acetat, Sulfat oder Hydrogensulfat steht.

3. Verfahren gemäß Anspruch 2, dadurch gekennzeichnet, daß Verbindungen der Formel (II) eingesetzt werden, in welchen
R¹ und R² für Methyl oder Ethyl stehen.

## Claims

1. Process for the preparation of cyanoimino-1,3-thiazolidine, characterized in that cysteamine salts of the formula (I) 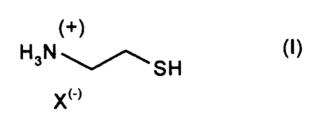 in which
X represents an acid radical
are reacted at temperatures of between 0 and 70°C in the presence of a diluent, in the presence of at least 2 equivalents of base and, if desired, in the presence of a protective-gas atmosphere with dialkyl N-cyanoimidocarbonates of the formula (II) 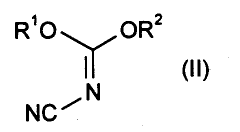 in which
R¹ and R² represent methyl, ethyl, n-propyl, isopropyl, n-butyl, isobutyl, sec-butyl or tert-butyl
to give the intermediate product of the formula (III) 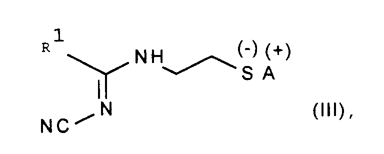 in which
R¹ has the abovementioned meaning, and
A⁽⁺⁾ represents a metal ion equivalent or an ammonium ion,
which is subsequently cyclized at a pH of from 8 to 9.5 to give cyanoimino-1,3-thiazolidine of the formula (IV)

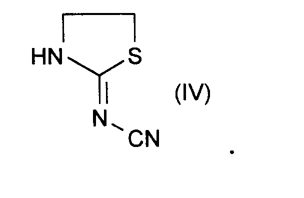

2. Process according to Claim 1, characterized in that compounds of the formula (I) are employed in which
X represents an acid radical from the group halogen, acetate, sulphate or hydrogen sulphate.

3. Process according to Claim 2, characterized in that compounds of the formula (II) are employed in which
R¹ and R² represent methyl or ethyl.

## Revendications

1. Procédé pour la préparation de la cyanimino-1,3-thiazolidine, caractérisé en ce qu'on fait réagir, à des températures entre 0 et 70°C, des sels de cystéamine répondant à la formule (I) 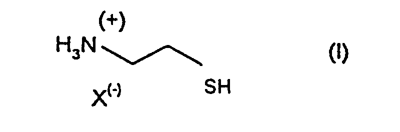 dans laquelle
X représente un reste d'acide,
en présence d'un diluant, en présence d'une base à au moins 2 équivalents et éventuellement en présence d'une atmosphère de gaz protecteur, avec des dialkyl-N-cyanimidocarbonates de formule (II) 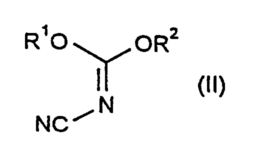 dans laquelle
R¹ et R² représentent un groupe méthyle, un groupe éthyle, un groupe n-propyle, un groupe i-propyle, un groupe n-butyle, un groupe i-butyle, un groupe sec.-butyle ou un groupe tert.-butyle, pour obtenir le produit intermédiaire de formule (III) 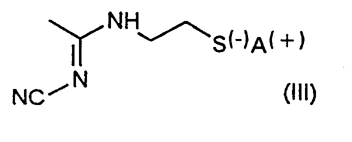
dans laquelle
R¹ possède la signification indiquée ci-dessus,
A⁽⁺⁾ représente un équivalent d'ion métallique ou un ion ammonium,
et ensuite, on cyclise à une valeur de pH de 8 à 9,5 pour obtenir la cyanimino-1,3-thiazolidine de formule (IV)

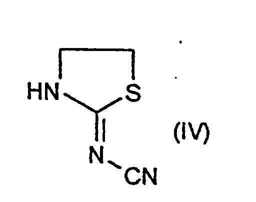

2. Procédé selon la revendication 1, caractérisé en ce qu'on met en oeuvre des composés de formule (I) dans lesquels
X représente un reste d'acide choisi parmi le groupe comprenant un atome d'halogène, un acétate, un sulfate ou un hydrogénosulfate.

3. Procédé selon la revendication 2, caractérisé en ce qu'on met en oeuvre des composés de formule (II) dans lesquels
R¹ et R² représentent un groupe méthyle ou un groupe éthyle.
